# EUROPEAN PATENT SPECIFICATION

(11) **EP 2 813 171 B1**
(45) Date of publication and mention of the grant of the patent: **22.11.2017**
(21) Application number: 12867973.5
(22) Date of filing: 19.12.2012
(51) Int. Cl.: A61B 1/00, A61B 5/00, G01N 21/47

(54) **BIO-OPTICAL MEASUREMENT DEVICE AND MEASUREMENT PROBE**
BIOOPTISCHE MESSVORRICHTUNG UND MESSSONDE
DISPOSITIF DE MESURE BIO-OPTIQUE ET SONDE DE MESURE

(30) Priority: 10.02.2012 US 201261597326 P
(43) Date of publication of application: 17.12.2014
(73) Proprietor: Olympus Corporation, Tokyo 192-8507 (JP)
(72) Inventor: KAMIMURA, Kenji, Hachioji-shi, Tokyo 192-8507 (JP)
(74) Representative: Schicker, Silvia
(86) International application number: PCT/JP2012/082941
(87) International publication number: WO 2013/118399

(56) References cited:
- JP-A- 2003 501 704
- JP-A- 2005 049 228
- JP-A- 2008 203 234
- JP-A- 2009 210 368
- US-B1- 6 850 656
- US-B1- 7 139 076

## Description

### Field

The present invention relates to a bio-optical measurement apparatus that measures optical properties of scattered light and to a measurement probe for measurement.

### Background

In recent years, there has been a known bio-optical measurement apparatus that irradiates body tissue with illumination light and that estimates the properties of the body tissue on the basis of a measurement value of detected light that is reflected or scattered by the body tissue. Such a bio-optical measurement apparatus is used in combination with an endoscope that observes an organ, such as a digestive organ. For example, there is a proposed bio-optical measurement apparatus that uses low-coherence enhanced backscattering (LEBS) technology that detects the properties of body tissue by irradiating the body tissue with white light that is low coherent light having a short spatial coherence length, from the distal end of an illumination fiber of a measurement probe and measuring, using multiple light-receiving fibers, the intensity distribution of scattered light at multiple angles (see Patent Literature 1).
Patent Literature 2 discloses a probe having two illumination fibers, which are disposed symmetrically in regard to collecting fibers.

### Citation List

### Patent Literature

Patent Literature 1: International Publication Pamphlet No. WO 2007/133684
Patent Literature 2: US 6,850,656 B1

### Summary

### Technical Problem

The above-described bio-optical measurement apparatus is usually inserted into the body cavity of a subject via a treatment instrument channel arranged in an endoscope apparatus; therefore, the diameter of the measurement probe needs to be reduced. Accordingly, the diameter of each fiber arranged in the measurement probe also needs to be reduced. However, if the diameter of an illumination fiber is reduced, it is difficult to irradiate body tissue with light having a sufficient intensity.

Accordingly, the present invention has been made in view of the foregoing and an object of the invention is to provide a measurement probe and a bio-optical measurement apparatus that allows irradiation of sufficient intensity light even if the diameter of the measurement probe is reduced.

### Solution to Problem

To solve the problem described above and achieve the object, a measurement probe according to the invention comprises the features according to claim 1. A preferable embodiment is provided in claim 2. In particular, the measurement probe is detachably connected to a bio-optical measurement apparatus which performs optical measurement on body tissue. The measurement probe includes two illumination fibers, and three or more light-receiving fibers. Distances between any of the light-receiving fibers and each of the illumination fibers are the same.

According to the measurement probe of the invention, the number of the illumination fibers is two, and the light-receiving fibers are arranged on a perpendicular bisector of a straight line connecting two of the illumination fibers.

A bio-optical measurement apparatus may include the measurement probe described above.

### Advantageous Effects of Invention

According to the present invention, in the measurement probe, the distances between any of the light-receiving fibers and each of the illumination fibers are the same. Accordingly, irradiation of sufficient intensity light can be achieved even if the diameter of the measurement probe is reduced.

### Brief Description of Drawings

FIG. 1 is a block diagram schematically illustrating the configuration of a bio-optical measurement apparatus according to an embodiment of the present invention.
FIG. 2 is a sectional view illustrating a distal end portion, cut in the longitudinal direction, of a measurement probe illustrated in FIG. 1.
FIG. 3 is a schematic diagram illustrating the distal end surface of the measurement probe illustrated in FIG. 2.
FIG. 4 is a schematic diagram illustrating a distal end surface of a measurement probe in a bio-optical measurement apparatus according to a first example which is useful for understanding the present invention.
FIG. 5 is a schematic diagram illustrating a distal end surface of a measurement probe in a bio-optical measurement apparatus according to a second example which is useful for understanding the present invention.
FIG. 6 is a sectional view illustrating a distal end portion, cut in the longitudinal direction, of a measurement probe in a bio-optical measurement apparatus according to a third example which is useful for understanding the present invention.
FIG. 7 is a sectional view illustrating the distal end surface of the measurement probe in the bio-optical measurement apparatus according to the third example.
FIG. 8 is a sectional view illustrating a distal end surface of a measurement probe in a bio-optical measurement apparatus according to a fourth example which is useful for understanding the present invention.

### Description of Embodiments

In the following, an embodiment of a bio-optical measurement apparatus and a measurement probe according to the present invention will be described in detail with reference to the accompanying drawings. The present invention is not limited to the embodiment. In the drawings, components that are identical to those in the embodiment are assigned the same reference numerals. The drawings used for the descriptions below are only schematic illustrations. The relationship between the thickness and the width of each member, the proportions of each member, and so on are different from those used in practice. The size or reduction in scale of elements may sometimes differ between the drawings.

### (Embodiment)

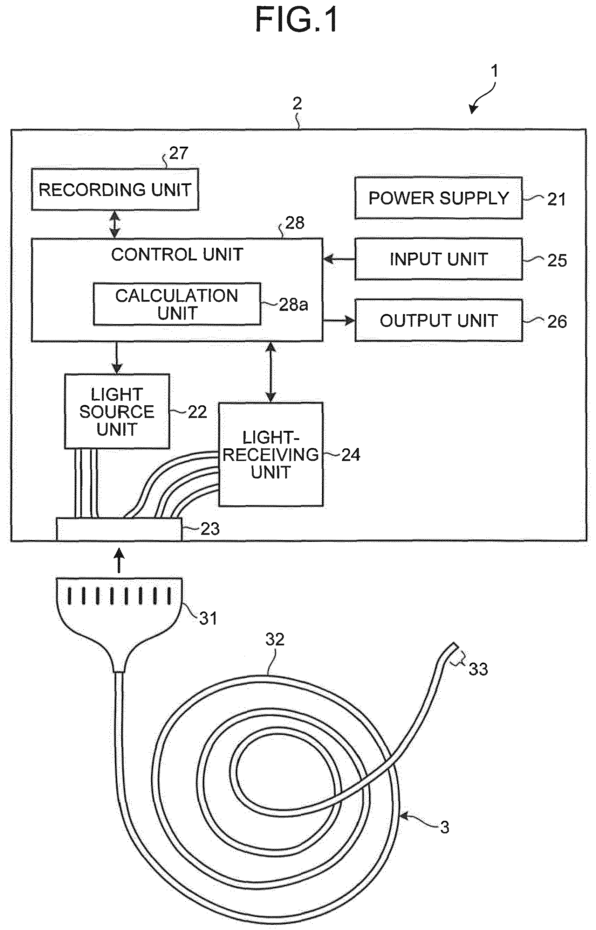

FIG. 1 is a block diagram schematically illustrating the configuration of a bio-optical measurement apparatus according to an embodiment of the present invention. As illustrated in FIG. 1, a bio-optical measurement apparatus 1 includes a main body unit 2 that performs optical measurement on a measurement object, such as body tissue that is a scatterer, and that detects the properties of a measurement object and, furthermore, the bio-optical measurement apparatus 1 includes a measurement probe 3 that is used for measurement and is inserted into a subject.

The main body unit 2 includes a power supply 21, a light source unit 22, a connecting unit 23, a light-receiving unit 24, an input unit 25, an output unit 26, a recording unit 27, and a control unit 28. The power supply 21 supplies electrical power to each component of the main body unit 2.

The light source unit 22 is implemented by using an incoherent light source, such as a white light emitting diode (LED), a xenon lamp, a tungsten lamp, or a halogen lamp, and by using, as necessary, a single lens or multiple lenses. The light source unit 22 supplies, to the measurement probe 3 via the connecting unit 23, incoherent light that has at least one spectral component that irradiates the measurement object with light.

The connecting unit 23 detachably connects the proximal end of the measurement probe 3 to the main body unit 2. The connecting unit 23 supplies light emitted from the light source unit 22 to the measurement probe 3 and outputs, to the light-receiving unit 24, scattered light that is output from the measurement probe 3. The connecting unit 23 outputs, to the control unit 28, information on the connection status of the measurement probe 3.

The light-receiving unit 24 receives scattered light, which is emitted from the measurement probe 3 and is scattered on the measurement object, and measures the scattered light. The light-receiving unit 24 is implemented by using multiple spectrometers. Specifically, the spectrometer in the light-receiving unit 24 is arranged in accordance with the number of light-receiving fibers of the measurement probe 3, which will be described later. The light-receiving unit 24 measures spectral components and intensity distribution of the scattered light incident from the measurement probe 3 and measures each wavelength. The light-receiving unit 24 outputs the measurement results to the control unit 28.

The input unit 25 is implemented by using, for example, a push button or touch panel switch; receives a start signal that instructs the bio-optical measurement apparatus 1 to start or an operation signal that instructs to operate various operations; and outputs the signals to the control unit 28.

The output unit 26 is implemented by using a display, such as a liquid crystal display or an organic electro luminescence (EL) display, and by using a speaker and outputs information on the various processes performed by the bio-optical measurement apparatus 1.

The recording unit 27 is implemented by using a volatile memory or a nonvolatile memory and records various programs used to operate the bio-optical measurement apparatus 1 and various kinds of data or parameters used for an optical measurement process. The recording unit 27 temporarily records information that is being processed by the bio-optical measurement apparatus 1. Furthermore, the recording unit 27 records the measurement results obtained by the bio-optical measurement apparatus 1. The recording unit 27 may also be configured by using a memory card mounted outside the bio-optical measurement apparatus 1.

The control unit 28 is configured by a central processing unit (CPU) or the like. The control unit 28 controls the flow of processes performed by each unit in the bio-optical measurement apparatus 1. The control unit 28 controls the operation of the bio-optical measurement apparatus 1 by transferring instruction information or data with respect to each unit in the bio-optical measurement apparatus 1. The control unit 28 records, in the recording unit 27, the measurement results obtained by the light-receiving unit 24. The control unit 28 includes a calculation unit 28a.

The calculation unit 28a performs multiple calculation processes on the basis of the measurement results obtained by the light-receiving unit 24 and calculates characteristic values related to the properties of the measurement object. The type of the characteristic values is set in accordance with, for example, an instruction signal received by the input unit 25.

The measurement probe 3 is implemented by using multiple optical fibers. Specifically, the measurement probe 3 is implemented by using illumination fibers that emit illumination light to the measurement object and by using multiple light-receiving fibers in which reflected light reflected by the measurement object and/or scattered light is incident at different angles. The measurement probe 3 includes a proximal end portion 31 that is detachably connected to the connecting unit 23 in the main body unit 2; a flexible portion 32 that has flexibility; and a distal end portion 33 through which light supplied from the light source unit 22 is emitted and reflected light reflected by the measurement object and/or scattered light enters.

The bio-optical measurement apparatus 1 having the above-described configuration is inserted into a subject via the treatment instrument channel arranged in the endoscope apparatus in the endoscope system; irradiates the measurement object with illumination light; and receives, by the light-receiving unit 24, the reflected light reflected by the measurement object and/or scattered light. Then, the calculation unit 28a measures, on the basis of the received light results output from the light-receiving unit 24, the properties of the measurement object.

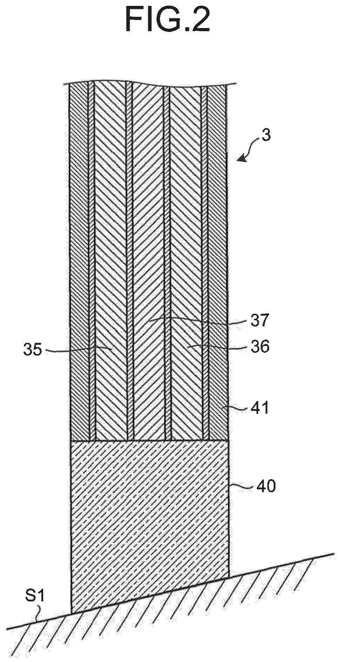

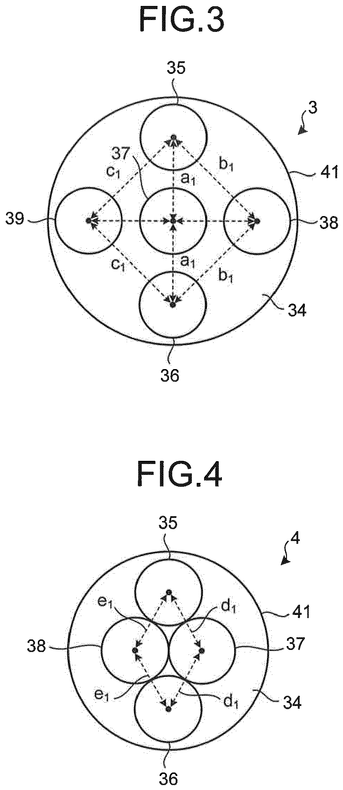

In the following, the measurement probe 3 illustrated in FIG. 1 will be described in detail. FIG. 2 is a sectional view illustrating a distal end portion, cut in the longitudinal direction, of the measurement probe 3 illustrated in FIG. 1. FIG. 3 is a schematic diagram illustrating a distal end surface 34 of the measurement probe illustrated in FIG. 2.

As illustrated in FIGS. 2 and 3, the measurement probe 3 includes a first illumination fiber 35, a second illumination fiber 36, a first light-receiving fiber 37, a second light-receiving fiber 38, a third light-receiving fiber 39, and an optical member 40. The sides of the first illumination fiber 35, the second illumination fiber 36, the first light-receiving fiber 37, the second light-receiving fiber 38, and the third light-receiving fiber 39 are covered by a covering layer 41 to block light and prevent damage.

The first illumination fiber 35 and the second illumination fiber 36 propagate illumination light supplied from the light source unit 22 to the distal end and irradiates measurement object S1 with light from the distal end.

Each of the first light-receiving fiber 37, the second light-receiving fiber 38, and the third light-receiving fiber 39 outputs, from the proximal end, reflected light that enters from each of the distal ends and that is reflected by the measurement object S1 and/or scattered light.

The optical member 40 fixes the distance between the first illumination fiber 35 and the measurement object S1 and the distance between the second illumination fiber 36 and the measurement object S1 such that light is irradiated in a state in which the spatial coherence length is reliably kept constant. Furthermore, the optical member 40 fixes the distance between the first light-receiving fiber 37 and the measurement object S1, the distance between the second light-receiving fiber 38 and the measurement object S1, and the distance between the third light-receiving fiber 39 and the measurement object S1 such that light having a predetermined scattering angle is stably received. Furthermore, because the surface of the measurement object S1 is flattened by the bottom surface of the optical member 40, measurements can be performed without being affected by the irregularities of the surface of the measurement object S1.

As illustrated in FIG. 3, in the measurement probe 3, the distances between any one of the light-receiving fibers and each of the illumination fibers are the same. Specifically, the first light-receiving fiber 37, the second light-receiving fiber 38, and the third light-receiving fiber 39 are arranged on the perpendicular bisector of the line connecting the center of the first illumination fiber 35 and the center of the second illumination fiber 36. For example, if the first light-receiving fiber 37 is taken as a reference, the distances to the first illumination fiber 35 and to the second illumination fiber 36 are the distance a₁. Furthermore, if the second light-receiving fiber 38 is taken as a reference, the distances to the first illumination fiber 35 and to the second illumination fiber 36 are the distance b₁. Furthermore, if the third light-receiving fiber 39 is taken as a reference, the distances to the first illumination fiber 35 and to the second illumination fiber 36 are the distance c₁.

In this way, in the measurement probe 3, the first illumination fiber 35 and the second illumination fiber 36 are arranged at positions where the distances between any of the light-receiving fibers and each of the illumination fibers are the same. Accordingly, because each of the scattering angles of scattered light received by each of the first light-receiving fiber 37, the second light-receiving fiber 38, and the third light-receiving fiber 39 is uniquely fixed, it is possible to receive an angular component, which is the same component as that obtained when using a single illumination fiber, having the light level increased by a factor of 2. Accordingly, light having a sufficient intensity can be irradiated and the diameter of the measurement probe can be reduced.

According to the embodiment of the present invention described above, in the measurement probe 3, if any one of the light-receiving fibers is taken as a reference, the first illumination fiber 35 and the second illumination fiber 36 are arranged such that the distances to each of the illumination fibers are the same. Accordingly, light having a sufficient intensity can be irradiated and the diameter of the measurement probe 3 can be reduced.

Furthermore, according to the embodiment of the present invention, in the measurement probe 3, the first illumination fiber 35 and the second illumination fiber 36 are arranged such that the distances between any of the light-receiving fibers and each of the illumination fibers are the same. Accordingly, it is possible to receive, without light received by each of the light-receiving fibers being interfered with, light that has the same properties and has the light level increased by a factor of 2, and thereby the SN ratio can be improved.

### (First Example)

In the embodiment, three light-receiving fibers are arranged; however, as illustrated in FIG. 4, in a first example which is useful for understanding the invention two light-receiving fibers are used. In such a case, in a measurement probe 4, the first illumination fiber 35 and the second illumination fiber 36 are arranged such that the distances (e₁, d₁) between any of the light-receiving fibers and each of the illumination fibers are the same. In such a case, the other light-receiving fiber may also be arranged on the perpendicular bisector of the line connecting the center of the first illumination fiber 35 and the center of the second illumination fiber 36. Furthermore, if a light-receiving fiber is arranged on the perpendicular bisector of the line connecting the center of the first illumination fiber 35 and the center of the second illumination fiber 36, it is possible to appropriately change the number of light-receiving fibers. Accordingly, light having a sufficient intensity can be irradiated and the diameter of the measurement probe 4 can be reduced.

### (Second Example)

In the following, a second example which is useful for understanding the present invention will be described. With a bio-optical measurement apparatus according to the second example, the configuration of a measurement probe differs from that in the first example described above. Accordingly, in the following, only the configuration of the measurement probe in the bio-optical measurement apparatus according to the second example will be described. Components that are identical to those in examples are assigned the same reference numerals.

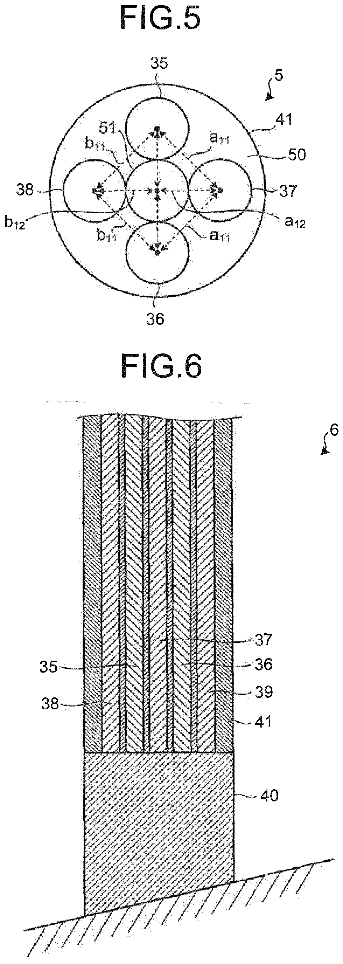

FIG. 5 is a schematic diagram illustrating a distal end surface 50 of a measurement probe 5 in the bio-optical measurement apparatus 1 according to the second example.

As illustrated in FIG. 5, the measurement probe 5 includes the first illumination fiber 35, the second illumination fiber 36, a third illumination fiber 51, the first light-receiving fiber 37, and the second light-receiving fiber 38. The third illumination fiber 51 has the same configuration as that of the first illumination fiber 35.

In the measurement probe 5, the distances between any of the light-receiving fibers and each of the illumination fibers are the same. Specifically, the first light-receiving fiber 37 and the second light-receiving fiber 38 are arranged on the perpendicular bisector of the line connecting the center of the first illumination fiber 35, the center of the second illumination fiber 36, and the third illumination fiber 51. For example, if the first light-receiving fiber 37 is taken as a reference, the distances to the first illumination fiber 35 and to the second illumination fiber 36 are the distance a₁₁ and the distance to the third illumination fiber 51 is a₁₂. Furthermore, if the second light-receiving fiber 38 is taken as a reference, the distances to the first illumination fiber 35 and to the second illumination fiber 36 are the distance b₁₁ and the distance to the third illumination fiber 51 is b₁₂.

In this way, in the measurement probe 5, the first illumination fiber 35, the second illumination fiber 36, and the third illumination fiber 51 are arranged such that the distances between any of the light-receiving fibers and each of the illumination fibers are substantially the same. Accordingly, because each of the scattering angles of scattered light received by each of the first light-receiving fiber 37 and the second light-receiving fiber 38 is uniquely fixed, it is possible to receive an angular component, which is the same component as that obtained when using a single illumination fiber, having the light level increased by a factor of 3. Accordingly, light having a sufficient intensity can be irradiated and the diameter of the measurement probe 5 can be reduced.

According to the second example described above, in the measurement probe 5, the first illumination fiber 35, the second illumination fiber 36, and the third illumination fiber 51 are arranged at positions where the distances between any of the light-receiving fibers and each of the illumination fibers are substantially the same. Accordingly, light having a sufficient intensity can be irradiated and the diameter of the measurement probe 5 can be reduced.

In the second example, three illumination fibers are arranged; however, the number of illumination fibers can be appropriately changed as long as the alignment of the illumination fibers is orthogonal to that of the light-receiving fibers. Specifically, the number of illumination fibers can be appropriately changed as long as the illumination fibers are arranged in a straight line.

### (Third Example)

In the following, a third example which is useful for understanding the present invention will be described. With a bio-optical measurement apparatus according to the third example, the configuration of a measurement probe differs from that in the examples described above. Accordingly, in the following, only the configuration of the measurement probe in the bio-optical measurement apparatus in the third example will be described. Components that are identical to those in the examples above are assigned the same reference numerals.

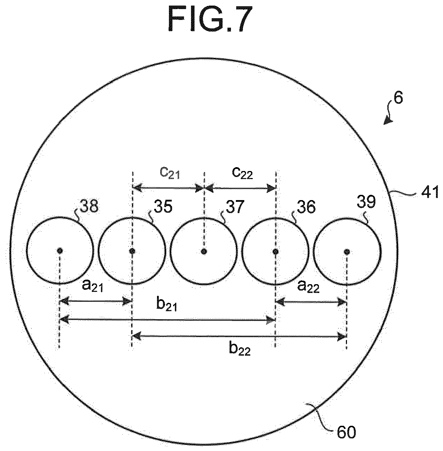

FIG. 6 is a sectional view illustrating a distal end portion, cut in the longitudinal direction, of a measurement probe 6 in the bio-optical measurement apparatus 1 according to the third example. FIG. 7 is a sectional view illustrating the distal end surface of the measurement probe 6 in the bio-optical measurement apparatus 1 according to the third example.

As illustrated in FIGS. 6 and 7, the measurement probe 6 includes the first illumination fiber 35, the second illumination fiber 36, the first light-receiving fiber 37, the second light-receiving fiber 38, the third light-receiving fiber 39, and the optical member 40. It is assumed that the sides of the first illumination fiber 35, the second illumination fiber 36, the first light-receiving fiber 37, the second light-receiving fiber 38, and the third light-receiving fiber 39 are covered by the covering layer 41 to block light and prevent damage. The distal end portions of the first illumination fiber 35, the second illumination fiber 36, the first light-receiving fiber 37, the second light-receiving fiber 38, and the third light-receiving fiber 39 are arranged in parallel each other. The first illumination fiber 35, the second illumination fiber 36, the first light-receiving fiber 37, the second light-receiving fiber 38, and the third light-receiving fiber 39 are alternately arranged in a straight line.

Furthermore, in the measurement probe 6, if a combination of light-receiving fibers is taken as a reference, the sums of the distances to each of the illumination fibers are equal. Specifically, if the second light-receiving fiber 38 is taken as a reference, the distance to the first illumination fiber 35 is the distance a₂₁ and the distance to the second illumination fiber 36 is the distance b₂₂. Furthermore, if the third light-receiving fiber 39 is taken as a reference, the distance to the first illumination fiber 35 is the distance b₂₂ and the distance to the second illumination fiber 36 is the distance a₂₂. Accordingly, if a combination of light-receiving fibers is taken as a reference, the sums of the distances to each of the illumination fibers are the same. Furthermore, if the first light-receiving fiber 37 is taken as a reference, the distance to the first illumination fiber 35 is the distance c₂₁ and the distance to the second illumination fiber 36 is the distance c₂₂.

In this way, in the measurement probe 6, if a combination of light-receiving fibers is taken as a reference, the first illumination fiber 35 and the second illumination fiber 36 are arranged at positions where the sums of the distances to each of the illumination fibers are the same. Accordingly, because each of the scattering angles of scattered light received by each of the first light-receiving fiber 37, the second light-receiving fiber 38, and the third light-receiving fiber 39 is uniquely fixed, it is possible to receive an angular component, which is the same component as that obtained when using a single illumination fiber, having the light level increased by a factor of 2. Accordingly, light having a sufficient intensity can be irradiated and the diameter of the measurement probe can be reduced.

According to the third example described above, in the measurement probe 6, if a combination of light-receiving fiber is taken as a reference with respect to two illumination fibers, the first illumination fiber 35 and the second illumination fiber 36 are arranged at positions where the sums of the distances to each of the illumination fibers are the same. Accordingly, light having a sufficient intensity can be irradiated and the diameter of the measurement probe 6 can be reduced.

Furthermore, according to the third example, because each fiber can be arranged two-dimensionally, the measurement probe 6 can be easily produced.

### (Fourth Example)

In the following, a fourth example which is useful for understanding the present invention will be described. With a bio-optical measurement apparatus according to the fourth example, the configuration of a measurement probe differs from that in the examples described above. Accordingly, in the following, only the configuration of the measurement probe in the bio-optical measurement apparatus according to the fourth example will be described. Components that are identical to those in the examples above are assigned the same reference numerals.

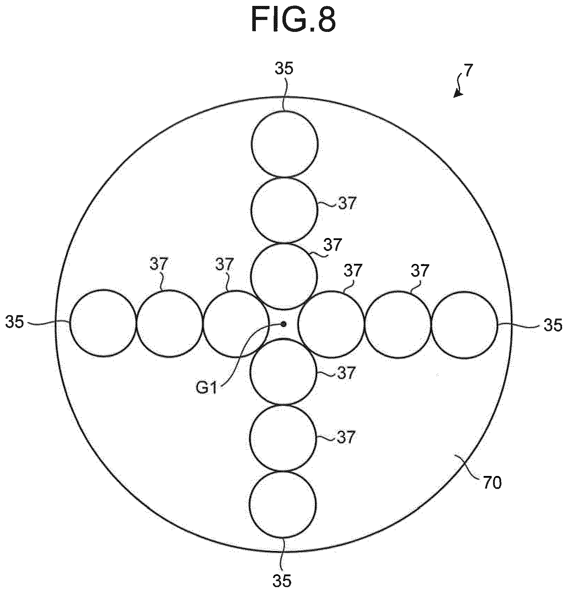

FIG. 8 is a sectional view illustrating a distal end surface 70 of a measurement probe 7 in the bio-optical measurement apparatus 1 according to the fourth example.

As illustrated in FIG. 8, the measurement probe 7 includes four first illumination fibers 35 and eight first light-receiving fibers 37. In the measurement probe 7, if the center of the measurement probe 7 is taken as a reference, the distances to each of the first illumination fibers 35 are the same. Specifically, each of the first illumination fibers 35 is arranged at a position on a circle with respect to the center of gravity G1 of the measurement probe 7. Furthermore, each of the first light-receiving fibers 37 is also arranged at a position on the circle with respect to the center of gravity G1 of the measurement probe 7.

In this way, in the measurement probe 7, the first illumination fibers 35 and the first light-receiving fibers 37 are arranged at positions on the circle with respect to the center of gravity G1 of the measurement probe 7. Accordingly, because each of the scattering angles of scattered light received by each of the first light-receiving fibers 37 is uniquely fixed, it is possible to receive an angular component, which is the same component as that obtained when using a single illumination fiber, having a light level increased by a factor of 4. Accordingly, light having a sufficient intensity can be irradiated and the diameter of the measurement probe can be reduced.

According to the fourth example described above, in the measurement probe 7, the first illumination fibers 35 and the first light-receiving fibers 37 are arranged at positions on a circle with respect to the center of gravity of the measurement probe 7. Accordingly, light having a sufficient intensity can be irradiated and the diameter of the measurement probe can be reduced.

### Reference Signs List

1 Bio-optical measurement apparatus
2 Main body unit
3, 4, 5, 6, 7 Measurement probe
21 Power supply
22 Light source unit
23 Connecting unit
24 Light-receiving unit
25 Input unit
26 Output unit
27 Recording unit
28a Calculation unit
28 Control unit
31 Proximal end portion
32 Flexible portion
33 Distal end portion
34 Distal end surface
35 First illumination fiber
36 Second illumination fiber
37 First light-receiving fiber
38 Second light-receiving fiber
39 Third light-receiving fiber
40 Optical member
41 Covering layer
51 Third illumination fiber

## Claims

1. A measurement probe (3) that is detachably connected to a bio-optical measurement apparatus (1) which performs optical measurement on body tissue, the measurement probe comprising:
only first and second illumination fibers (35, 36) configured to irradiate the body tissue with low coherent light having a short spatial coherence length; and
three or more light-receiving fibers (37, 38, 39) configured to receive scattered light at multiple angles,
**characterized in that** all light-receiving fibers (37, 38, 39) of the measurement probe are arranged on a perpendicular bisector of a line segment connecting centers of the first and second illumination fibers (35, 36) at a distal end surface of the measurement probe (3), such that a distance between the first illumination fiber (35) and any one of the light-receiving fibers (37, 38, 39) is equal to a distance between the second illumination fiber (36) and the any one of the light-receiving fibers (37, 38, 39).

2. A bio-optical measurement apparatus that includes the measurement probe (3) according to claim 1.

## Patentansprüche

1. Messsonde (3), die abnehmbar mit einem bio-optischen Messgerät (1) verbunden ist, das eine optische Messung an Körpergewebe ausführt, wobei die Messsonde Folgendes umfasst:
nur erste und zweite Beleuchtungsfasern (35, 36), die konfiguriert sind, um das Körpergewebe mit schwach kohärentem Licht zu bestrahlen, das eine geringe räumliche Kohärenzlänge aufweist; und
drei oder mehrere Licht empfangende Fasern (37, 38, 39), die konfiguriert sind, um gestreutes Licht in mehreren Winkeln zu empfangen,
**dadurch gekennzeichnet, dass** alle Licht empfangenden Fasern (37, 38, 39) der Messsonde auf einer rechtwinkligen Halbierungslinie einer Strecke angeordnet sind, welche die Mittelpunkte der ersten und zweiten Beleuchtungsfasern (35, 36) an einer distalen Endfläche der Messsonde (3) verbindet, so dass ein Abstand zwischen der ersten Beleuchtungsfaser (35) und einer der Licht empfangenden Fasern (37, 38, 39) gleich einem Abstand zwischen der zweiten Beleuchtungsfaser (36) und einer der Licht empfangenden Fasern (37, 38, 39) ist.

2. Bio-optisches Messgerät, das die Messsonde (3) nach Anspruch 1 umfasst.

## Revendications

1. Sonde de mesure (3) qui est connectée de manière détachable à un appareil de mesure bio-optique (1) qui réalise une mesure optique sur un tissu de corps, la sonde de mesure comprenant :
uniquement des première et seconde fibres d'éclairement (35, 36) configurées pour irradier le tissu de corps avec une faible lumière cohérente ayant une longueur de cohérence spatiale courte ; et
au moins trois fibres de réception de lumière (37, 38, 39) configurées pour recevoir une lumière diffusée à de multiples angles,
**caractérisée par le fait que** toutes les fibres de réception de lumière (37, 38, 39) de la sonde de mesure sont agencées sur une bisectrice perpendiculaire d'un segment de ligne reliant les centres des première et seconde fibres d'éclairement (35, 36) au niveau d'une surface d'extrémité distale de la sonde de mesure (3), de telle sorte qu'une distance entre la première fibre d'éclairement (35) et l'une quelconque des fibres de réception de lumière (37, 38, 39) est égale à une distance entre la seconde fibre d'éclairement (36) et ladite quelconque des fibres de réception de lumière (37, 38, 39).

2. Appareil de mesure bio-optique qui comprend la sonde de mesure (3) selon la revendication 1.
